# EUROPEAN PATENT SPECIFICATION

(11) **EP 1 503 680 B1**
(45) Date of publication and mention of the grant of the patent: **23.12.2009**
(21) Application number: 03736492.4
(22) Date of filing: 24.04.2003
(51) Int. Cl.: A61B 17/34

(54) **INTRODUCER SEAL ASSEMBLY**
EINFÜHRDICHTUNGSANORDNUNG
JOINT D'ETANCHEITE POUR INTRODUCTEUR

(30) Priority: 10.05.2002 US 379651 P; 04.10.2002 US 264556
(43) Date of publication of application: 09.02.2005
(73) Proprietor: Tyco Healthcare Group LP, Norwalk, Connecticut 06856 (US)
(72) Inventor: SMITH, Robert, C., Cheshire, CT 06410 (US)
(74) Representative: HOFFMANN EITLE
(86) International application number: PCT/US2003/012894
(87) International publication number: WO 2003/094760

(56) References cited:
- WO-A-02/41795
- WO-A-97/42991
- WO-A-98/53865
- US-A- 5 342 315
- US-A- 5 720 759
- US-A- 5 792 113

## Description

### BACKGROUND

### 1. Technical Field

The present disclosure relates to valve system adapted to permit the introduction of surgical instrumentation into a patient's body. In particular, the present disclosure relates to a valve system for use with an introducer which is intended for insertion into a patient's body, and to receive an instrument in sealing engagement therewith.

### 2. Description of the Related Art

U.S. Patent No. 5,342,315 discloses a trocar assembly that includes a trocar tube and a housing adjoining a proximal end portion of the trocar tube that defines a chamber. An elastomeric seal member is positioned within the chamber for sealing the chamber as an elongate instrument passes through an opening formed in the seal member. A seal/protector assembly is positioned within the chamber proximally of the seal member in facing relationship thereto. The protector assembly includes at least two axially aligned protector members, each of which has an annular collar portion and at least one leaf portion formed integrally with the collar portion so as to form a living hinge portion. The leaf portions define an opening therethrough in axial alignment with the opening in the seal member. Insertion of an elongate instrument into contact with the leaf portions causes them to pivot distally, increasing the size of the opening which they define, and permitting the instrument to pass therethrough as well as through the opening in the seal member without causing damage to the seal member.

International Publication No. WO 97/42991 discloses an adjustable pnemostasis valve and method for providing a fluid seal about surgical instruments inserted therethrough which may have a variety of cross-sectional sizes. The valve comprises a first valve member having an opening for receiving a surgical instrument, and a flexible membrane defining an aperture substantially aligned with the opening in the first valve member. A second valve member is coupled to the flexible membrane for moving the membrane relative to the first valve member. As the membrane is moved by the second valve member, the size of the aperture varies so that the membrane can accommodate surgical instruments of various sizes. The pre-amble of claim 1 is based on this document.

Minimally invasive and laparoscopic procedures generally require that any instrumentation inserted into the body is sealed, i. e., provisions must be made to ensure that gases and/or fluids do not enter or exit the body through an endoscopic incision, such as, for example in surgical procedures where the surgical region is insufflated. For such procedures, the introduction of a tube into anatomical cavities, such as the peritoneal cavity, is usually accomplished by use of a system incorporating a trocar and cannula assembly. Since the cannula is in direct communication with the interior of the peritoneal cavity, insertion of the cannula into an opening in the patient's body to reach the inner abdominal cavity should be adapted to maintain a fluid tight interface between the abdominal cavity and the outside atmosphere. In view of the need to maintain the atmospheric integrity of the inner area of the cavity, a seal assembly for a cannula, which permits introduction of a wide range of surgical instrumentation and maintains the atmospheric integrity of the inner area of the cavity is desirable. In this regard, there have been a number of attempts in the prior art to achieve such sealing requirements. A difficulty encountered with conventional seal assemblies, however, is the inability of accommodating the wide range of sizes of instrumentation. In addition, angulation and/or manipulation of instrumentation within the cannula often present difficulties with respect to maintaining seal integrity.

### SUMMARY

The present invention provides a seal assembly for an access device as defined in claim 1.

Accordingly, the present disclosure provides a seal assembly which will allow a surgeon to efficaciously utilize instruments of varying diameter in a surgical procedure. This seal assembly obviates the need for multiple adapters to accommodate instruments of varying diameter by providing an apertured resilient seal member which is mounted in a gimbal-like assembly, thereby facilitating alignment of the instrument with the aperture of the seal member.

In a preferred embodiment, a seal assembly for use with an access device includes a seal housing defining a central longitudinal axis. The seal housing includes an inner wall and an outer wall. The inner wall defines a longitudinal opening to permit passage of instrumentation through the seal housing. A gimbal mount is at least partially accommodated within a space defined between the inner wall and the outer wall of the seal housing. The gimbal mount includes a seal member defining an aperture for substantial sealed reception of a surgical instrument. The gimbal mount is adapted for angular movement relative to the central longitudinal axis upon angulation of the surgical instrument while substantially maintaining the sealed reception of the surgical instrument. The gimbal mount preferably defines a general hemispherical configuration.

The seal housing may include a skirt seal which is positioned adjacent the gimbal mount and adapted to minimize passage of fluids through the seal housing. The skirt seal may extend to contact the gimbal mount, and bias the gimbal mount in a general proximal direction. The skirt seal is dimensioned and configured to bias the gimbal mount against the inner wall of the seal housing. The inner wall of the seal housing defines a distal arcuate surface in contacting relation with a corresponding inner arcuate surface of the gimbal mount.

The preferred seal member includes a resilient member and a protective layer juxtaposed relative to the resilient member. The protective layer of the seal member extends at least partially within the aperture to protect portions of the seal member defining the aperture during passage of the surgical instrument.

### The protective layer may include a fabric material.

The seal housing is adapted to be detachably mounted to a cannula assembly for providing a substantially fluid-tight seal when the instrument is inserted into the seal assembly and through the cannula assembly.

In an alternate embodiment, the seal assembly for use with an access device includes a seal housing defining a central longitudinal axis and having proximal and distal ends. The seal housing includes an inner wall defining an opening to permit passage of instrumentation through the seal housing. A gimbal mount is disposed within the seal housing. The gimbal mount is adapted for angular movement within the seal housing about an axis of rotation. The gimbal mount includes a seal defining an aperture for sealed reception of a surgical instrument. A skirt member is engageable with a peripheral portion of the gimbal mount, and is dimensioned to bias the gimbal mount in a proximal direction against the seal housing. The seal housing defines a distal angulating surface which is in contacting relation with the gimbal mount Preferably, the gimbal mount defines an interior surface corresponding to the distal angulating surface of the seal housing, and in contacting relation therewith. The interior surface traverses the distal angulating surface upon angular movement of the gimbal mount. The gimbal mount may also define a general hemispherical configuration.

In another embodiment, the seal assembly for use with an access device includes a seal housing defining a central longitudinal axis and a longitudinal passageway for permitting passage of a surgical instrument, and a generally hemispherical seal element disposed within the seal housing. The seal element defines a seal axis and an aperture for sealed reception of the surgical instrument. The seal element is adapted for angular movement within the seal housing to accommodate angular movement of the surgical instrument whereby the seal axis intersects the central longitudinal axis of the seal housing.

The seal assembly is adapted to be associated with a cannula assembly. The cannula assembly typically includes a tubular cannula and a cannula housing within which is positioned a cannula seal assembly. The cannula seal assembly typically provides structure which is adapted to provide a fluid-tight seal in the absence of a surgical instrument Suitable cannula seal assemblies include a spring loaded flapper valve, a trumpet valve, a duck bill valve, or the like. The seal assembly of the invention may be associated with the cannula housing by any suitable means, e.g., a bayonet lock.

In use, the seal assembly may be associated with a cannula assembly at any point the surgeon desires flexibility in the instrument sizes he may utilize therethrough. Thus, for example, if the surgeon is utilizing a 15 mm cannula assembly in an endoscopic surgical procedure and determines that it would be advantageous to have the flexibility to use instruments ranging in size from 5 to 15 mm through that cannula assembly, the seal assembly may be secured to the cannula assembly. Thereafter, instruments ranging in diameter from 5 to 15 mm may be efficaciously introduced therethrough. The cylindrical guide wall guides the instrument toward the aperture of the resilient seal member. The gimbal mount angularly repositions itself with respect to the housing in response to the insertion and manipulation of the instrument.

The movement of the gimbal mount relative to the housing which is accommodated by the gimbal-like structure also facilitates seal maintenance once an instrument is being used within the body cavity. In particular, as an instrument is manipulated, the resilient seal member radially and transversely repositions itself through movement of the gimbal mount relative to the housing, thereby ensuring that the resilient seal member maintains a fluid-tight seal around the instrument shaft.

### BRIEF DESCRIPTION OF THE DRAWINGS

The foregoing features of the present disclosure will become more readily apparent and will be better understood by referring to the following detailed description of preferred embodiments, which are described hereinbelow with reference to the drawings wherein:
FIGS. 1 - 2 are perspective views of a cannula assembly and a seal assembly in accordance with the principles of the present disclosure;
FIG 3 is a perspective view with parts separated of the cannula and seal assemblies of FIG 1;
FIG 4 is a side cross-sectional view of the cannula and seal assemblies;
FIGS. 5 - 6 are top and bottom perspective views of the gimbal mount of the seal assembly;
FIGS. 7 - 8 are cross-sectional views of the gimbal mount;
FIG. 9 is a perspective view illustrating the components of the gimbal mount;
FIGS. 10 - 12 are perspective views illustrating the range of movement of the gimbal mount within the seal housing;
FIG 13 is a view illustrating the cannula assembly and seal assembly accessing an internal cavity with an instrument introduced therein; and
FIG 14 is a side cross-sectional view of the cannula and seal assemblies illustrating a range of movement of the surgical instrument.

### DETAILED DESCRIPTION OF PREFERRED EMBODIMENTS

The seal assembly of the present disclosure, either alone or in combination with a seal system internal to a cannula assembly, provides a substantial seal between a body cavity of a patient and the outside atmosphere before, during and after insertion of an instrument through the cannula assembly. Moreover, the seal assembly of the present invention is capable of accommodating instruments of varying diameters, e.g., from 5 mm to 15 mm, by providing a gas tight seal with each instrument when inserted. The flexibility of the present seal assembly greatly facilitates endoscopic surgery where a variety of instruments having differing diameters are often needed during a single surgical procedure.

The seal assembly contemplates the introduction and manipulation of various types of instrumentation adapted for insertion through a trocar and/or cannula assembly while maintaining a fluid tight interface about the instrumentation to preserve the atmospheric integrity of a surgical procedure from gas and/or fluid leakage. Specifically, the seal assembly accommodates angular manipulation of the surgical instrument relative to the seal axis. This feature of the present disclosure desirably minimizes the entry and exit of gases and/or fluids to/from the body cavity. Examples of instrumentation include clip appliers, graspers, dissectors, retractors, staplers, laser probes, photographic devices, endoscopes and laproscopes, tubes, and the like. Such instruments will be collectively referred to herein as "instruments or instrumentation".

In the following description, as is traditional the term "proximal" refers to the portion of the instrument closest to the operator while the term "distal" refers to the portion of the instrument remote from the operator.

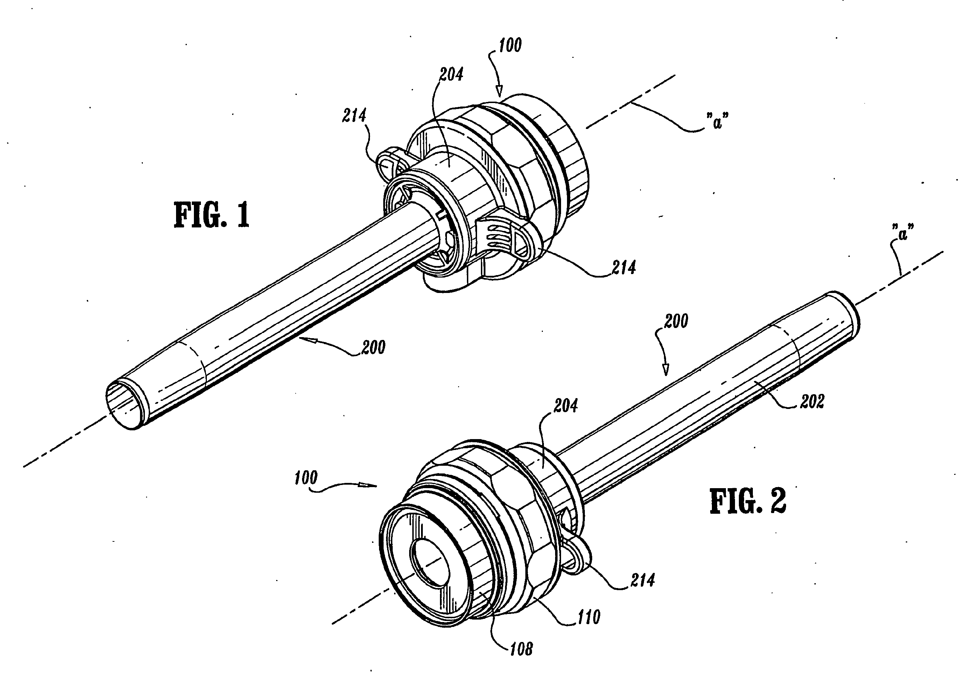

Referring now to the drawings, in which like reference numerals identify identical or substantially similar parts throughout the several views, FIGS. 1-2 illustrate the seal assembly 100 of the present disclosure mounted to cannula assembly 200. Cannula assembly 200 may be any conventional cannula suitable for the intended purpose of accessing a body cavity and permit introduction of instruments therethrough. Cannula assembly 200 is particularly adapted for use in laparoscopic surgery where the peritoneal cavity is insufflated with a suitable gas, e.g., CO₂, to raise the cavity wall from the internal organs therein. Cannula assembly 200 is typically used with an obturator assembly (not shown) which is a sharp pointed instrument positionable within the passageway of the cannula assembly 200. The obturator assembly is utilized to penetrate the abdominal wall and then subsequently removed from the cannula assembly to permit introduction of the surgical instrumentation utilized to perform the procedure.

Cannula assembly 200 includes cannula sleeve 202 and cannula housing 204 mounted to an end of the sleeve 202. Cannula sleeve 202 defines a longitudinal axis "a" extending along the length of sleeve 202. Sleeve 202 further defines an internal longitudinal passage dimensioned to permit passage of surgical instrumentation. Sleeve 202 may be formed of stainless steel or other rigid materials such as a polymeric material or the like. Sleeve 202 may be clear or opaque. The diameter of sleeve 202 may vary, but typically ranges from 10 to 15 mm for use with the seal assembly 100 of the present disclosure.

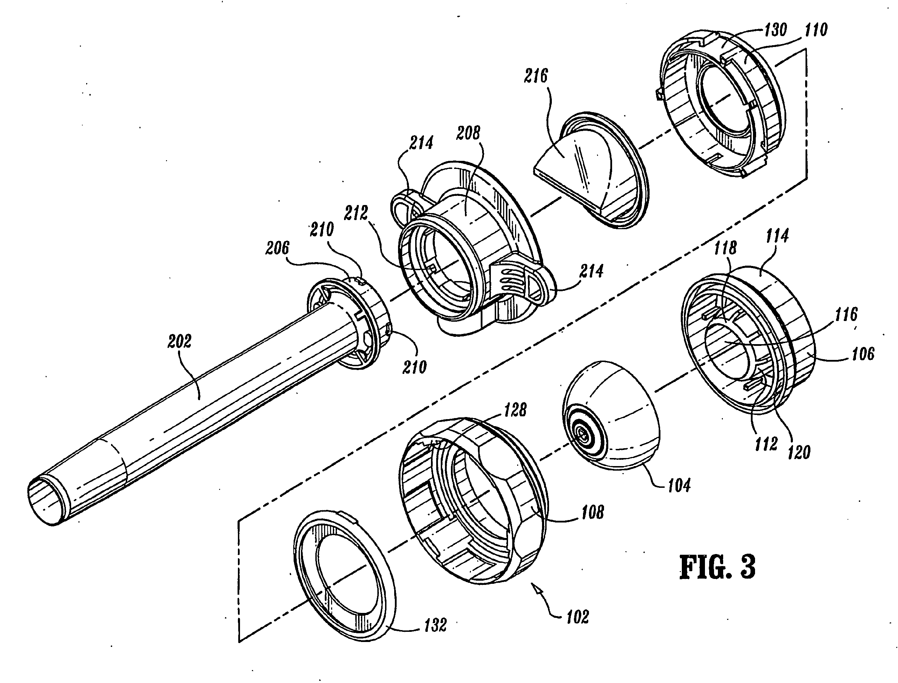

Cannula housing 204 includes two components, specifically, housing flange 206 which is attached to the proximal end of cannula sleeve 202 and main housing 208 as shown in FIGS. 3-4. Main housing 208 is connectable to housing flange 206 through a bayonet coupling consisting of radially spaced tongues 210 on the exterior of housing flange 206 and corresponding recesses 212 within the interior of main housing 208. Tongues 210 are receivable within recesses 212. Thereafter, housing flange 206 and main housing 208 are rotated to securely lock the tongues 210 within the recesses 212. Other conventional means, e.g., a snap fit, ultrasonic welding or any other means envisioned by one skilled in the art including, e.g., adhesive means, may be incorporated to connect housing flange 206 and main housing 208. Main housing 208 further includes diametrically opposed housing grips 214 dimensioned and arranged for gripping engagement by the fingers of the user. Although shown and described as two components, cannula housing 204 may be a single component and attached to cannula sleeve 202 by any of the aforementioned means.

With reference to FIG. 3, in conjunction with FIGS. 1-2, cannula housing 204 further includes duck bill or zero closure valve 216 which tapers distally and inwardly to a sealed configuration as shown in the figure. Valve 216 opens to permit passage of the surgical instrumentation and closes in the absence of the instrumentation. Valve 216 is preferably adapted to close upon exposure to the forces exerted by the insufflation gases in the internal cavity. Other zero closure valves are also contemplated including single or multiple slit valve arrangements, trumpet valves, flapper valves, etc.

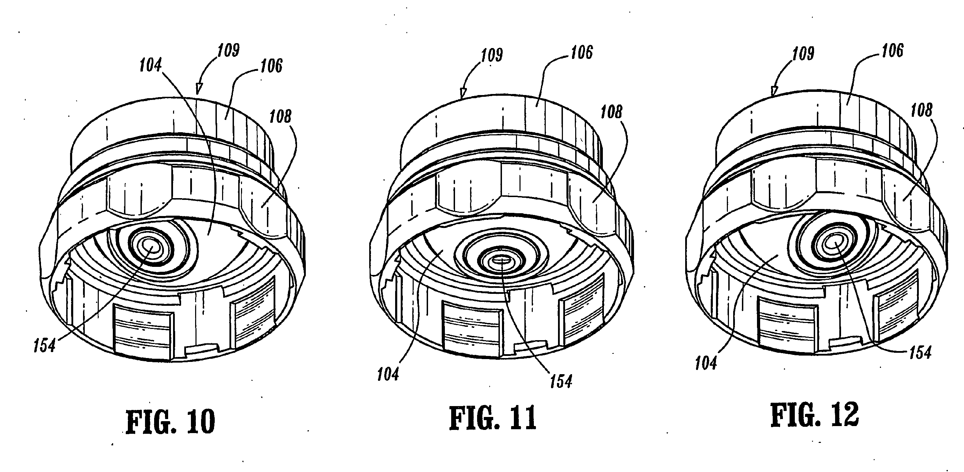

Referring now to FIGS. 3 - 4, in conjunction with FIGS. 1-2, seal assembly 100 will be discussed in detail. Seal assembly 100 includes seal housing, generally identified as reference numeral 102, and gimbal mount 104 which is disposed within the seal housing 102. Seal housing 102 houses the sealing components of the assembly and defines the outer valve or seal body of the seal assembly 100. Seal housing 102 defines central seal housing axis "b" which is preferably parallel to the axis "a" of cannula sleeve 202 and, more specifically, coincident with the axis "a" of the cannula. Seal housing 102 incorporates three housing components, namely, proximal, distal and inner housing components 106, 108, 110, respectively, which, when assembled together, form the seal housing 102. Assembly of housing components 106, 108, 110 may be affected by any of the aforementioned connection means discussed with respect to cannula housing 204. Further, seal housing 102 may be considered as having an upper housing portion 109 formed by components 106 108, as shown separately in FIGS. 10-12, and a detachable lower housing portion formed by component 110.

Proximal housing component 106 defines inner guide wall 112 and outer wall 114 disposed radially outwardly of the inner guide wall 112. Inner guide wall 112 defines central passage 116 which is dimensioned to receive a surgical instrument and laterally confine the instrument within seal housing 102. Inner guide wall 112 is generally cylindrical in configuration and terminates in a distal arcuate surface 118. Outer wall 114 defines first and second annular recesses 120, 122 adjacent its distal end. Recesses 120, 122 receive corresponding structure, e.g., annular lips 124, 126 of distal housing component 108 to facilitate connection of the two components. As appreciated, proximal housing component 106 may also incorporate locking tabs which engage corresponding structure of distal housing component 108 upon relative rotation of the components 106, 108 to securely connect the components.

Inner housing component 110 is disposed within the interior of distal housing component 108 and securely connectable to the distal housing component 108 through a bayonet coupling. Such coupling includes radially spaced tongues 128 which depend radially inwardly to be received within correspondingly arranged grooves or recesses 130 on the exterior of inner housing component 110. Coupling of distal and inner housing components 108, 110 is thereby affected through simple rotation of the components.

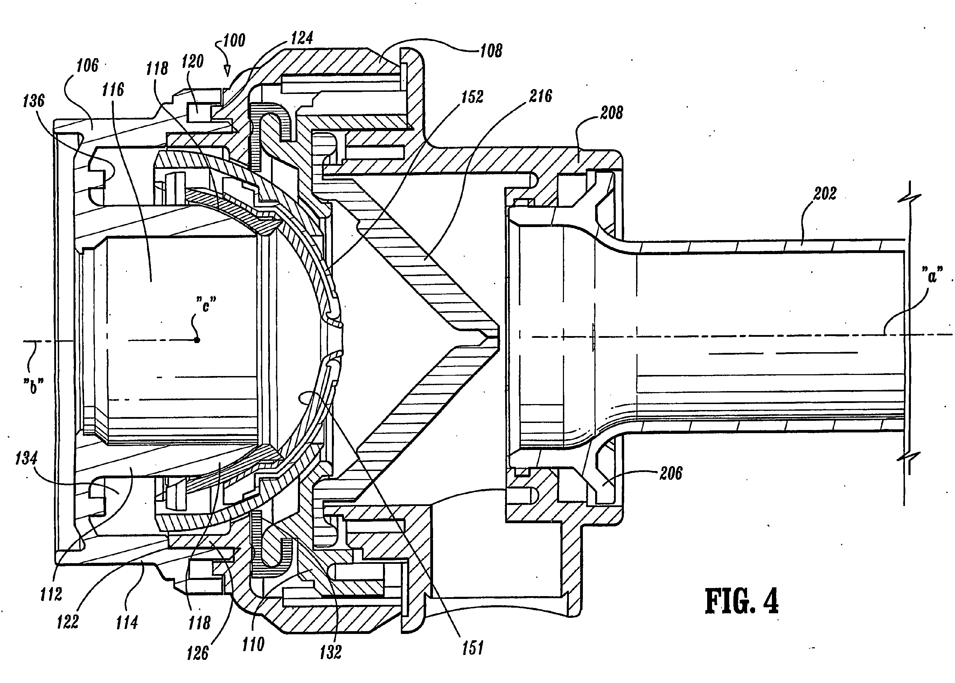

With continued reference to FIGS. 3 and 4, seal assembly 100 further includes skirt seal 132 mounted about the proximal end of inner housing component 110 or on the upper surface of the inner housing component (constituting a lower component) of the seal housing. Skirt seal 132 functions in minimizing the loss of insufflation gases through seal assembly 102. Skirt seal 132 also engages gimbal mount 104'and serves to bias the gimbal mount in a proximal direction against inner guide wall 112 of proximal housing 106 as will be discussed. Skirt seal 132 is preferably fabricated from a suitable elastomeric material or the like to provide a spring-like characteristic sufficient to appropriately bias gimbal mount 104.

With particular reference to FIG 4, gimbal mount 104 is accommodated within an annular space 134 defined between inner and outer walls 112, 114 of proximal housing component 106. Gimbal mount 104 is mounted in a manner which permits angulation of the gimbal mount 104 relative to seal axis "b". Specifically, gimbal mount 104 is free to angulate about an axis or center of rotation "c" through a range of motion defined within the confines of annular space 134. An annular stop 136 may extend within annular space 134. Annular stop 136 is positioned to limit the degree of angulation of gimbal mount 104 if desired. The range of movement of gimbal mount 104 will be discussed in greater detail hereinbelow.

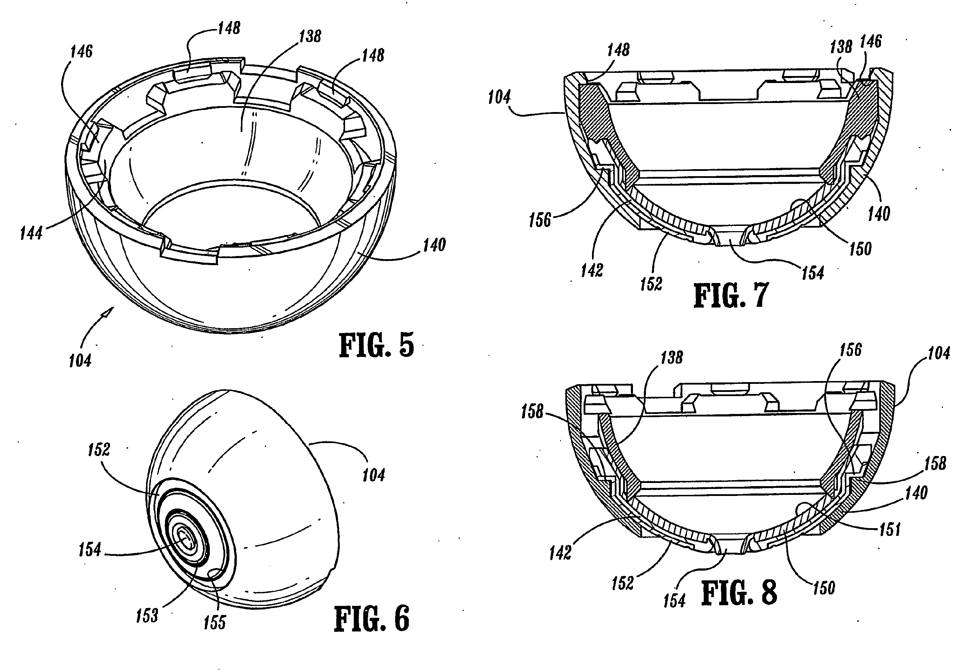

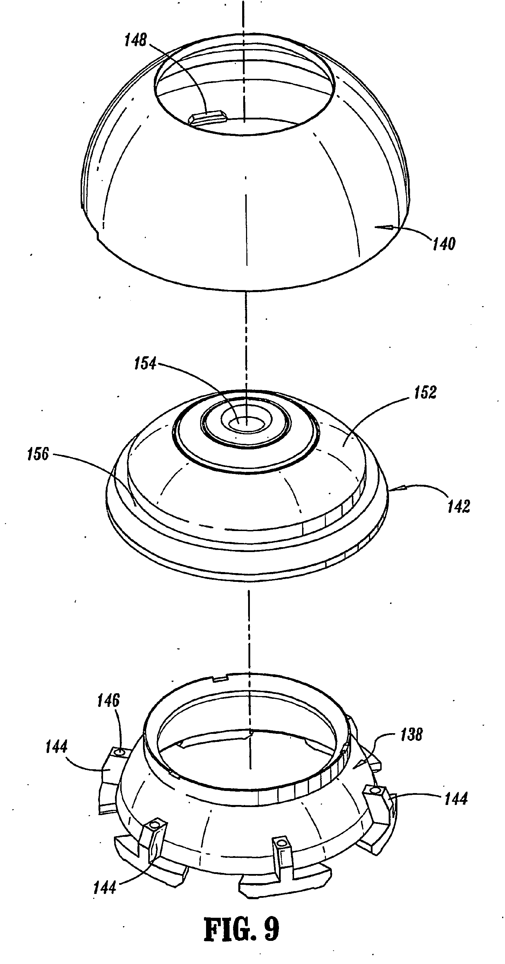

Referring now to FIGS. 5 - 9, in conjunction with FIG. 4, the components of gimbal mount 104 will be discussed in further detail. Gimbal mount 104 includes first and second gimbal housings 138, 140 and resilient seal member 142 which is mounted between the housings 138, 140. In a preferred arrangement, first and second gimbal housings 138, 140 and seal member 142 each define a general hemispherical configuration as shown. First gimbal housing 138 is preferably seated within second gimbal housing 140 and secured to the second gimbal housing 140 through a snap fit connection or the like. Preferably, first gimbal housing 138 includes a plurality of mounting legs 144 radially spaced about the outer periphery of the housing component 134. Legs 144 define locking surfaces 146 which extend in general transverse relation to the axis "b" of seal assembly 200. Similarly, second gimbal housing 140 includes a plurality of corresponding locking detents 148 spaced about the interior of the housing 140. Upon insertion of first gimbal housing 138 within second gimbal housing 140, mounting legs 144 slide along locking detents 148 whereby upon clearing the detents 148, locking surfaces 146 of the mounting legs 146 securely engage the locking detents 148 to fix first gimbal housing 138 within second gimbal housing 140 and securing resilient seal member 142 between the components in sandwiched relation. As appreciated, first gimbal housing 138 may be sufficiently resilient to deflect upon insertion to permit mounting legs 144 to clear locking detents 148 and return to their initial position to engage the detents 148.

As mentioned hereinabove, seal member 142 of gimbal mount 104 is secured in interposed relation between first and second gimbal housings 138, 140. Seal member 142 preferably comprises a resilient center material (e.g., polyisoprene or natural rubber) with first and second layers of fabric 150, 152 impregnated on the respective proximal and distal surfaces of the resilient center material. Fabric may be of any suitable fabric for example, a SPANDEX material containing about 20% LYCRA and about 80% NYLON available from Milliken. A suitable seal member or seal type is disclosed in commonly assigned U.S. Patent No. 6,482,181, filed November 24, 1999. Seal member 142 defines central aperture 154 for sealed reception of a surgical instrument In a preferred arrangement, first layer 150 is arranged to extend or overlap into aperture 154. In this manner, the fabric (which is stronger relative to the resilient material) is positioned to engage the surgical instrument upon passage through aperture 154 of seal member 142 thereby protecting the resilient material defining the aperture. This advantageously minimizes the potential of piercing, penetrating or tearing of the resilient material by the instrument. Alternatively, an additional layer of fabric 151 on the proximal surface of seal member 142 may be superposed and arranged to drape within aperture 154. Seal member 142 includes an annular depression 156 on its distal surface, i.e., within second layer 152 of fabric. Depression 156 receives ledge 158 of second gimbal housing 140 to facilitate fixation of seal member 142 between first and second gimbal housings 138, 140.

Although seal member 142 is disclosed as an impregnated fabric arrangement, it is appreciated that other seal types may be used and still achieve the objectives of the present disclosure. Further, FIG. 6 illustrates annular depressions 153, 155 which have been pressed by a molding tool into layer 153. One or more similar depressions may be pressed into layer 150 to assist positioning of fabric during manufacture of seal member 142.

With reference now to FIGS. 10-12, in conjunction with FIG. 4, gimbal mount 104 is free to move within the annular space 134 defined between inner and outer walls 112,114 to permit angulation of the instrument relative to the seal axis "b" while still maintaining a seal thereabout. Specifically, gimbal mount 104 is adapted for swiveling movement about a center of rotation "c" which is coincident with the axis of seal assembly 100. In this regard, the axis of the aperture 154 of seal member 142 intersects the axis "b" of the seal assembly 100 during angulation of the instrument. During angulation, gimbal mount 104 is only in contact with seal housing 102 along distal arcuate surface 118 of proximal housing 106 as well as along skirt seal 132. Specifically, the arcuate inner surface of first gimbal housing 13 8 rides along distal arcuate surface 118 of inner wall 112 in contacting relation therewith (under the bearing influence of skirt seal 132) to permit gimbal mount 104 to swivel within seal housing 102. Preferably, there is no other contact of gimbal mount 104 with any of the other components of seal housing, which thereby substantially minimizes resistance to the angulating movement A lubricant may be provided between distal arcuate surface 118 and the inner surface of first gimbal housing 138 to facilitate angulation.

In a preferred arrangement, gimbal mount 104 may angulate or rotate through an angle inclusive of about 25°, more preferably about 22.5° relative to seal axis "b". Annular stop 136 may further restrict angulation by a couple of degrees of movement to be inclusive of an angle of about 19° relative to axis "b".

Seal assembly 100 may be associated with, or joined to, cannula assembly 200 in a variety of ways. In a preferred embodiment, seal housing 102 of seal assembly 100 and cannula housing 204 of cannula assembly 200 are adapted to detachably engage each other, e.g., through a bayonet lock or like mechanical means. As previously discussed, proximal and distal housing components 106, 108 may define an upper housing component 109 which is mountable directly to cannula assembly 200. Alternatively, inner housing portion 110 which defines a lower housing component may be directly mounted to cannula assembly 200 independent of the upper housing component 109. Specifically, the lower housing component 110 which houses gimbal mount 104 may be mounted to cannula assembly independent of the remaining housing components. The upper housing may then be mounted to lower housing or cannula assembly 200 as needed. Even further, upper housing component 109 may be mounted to cannula assembly 200 without lower housing component 110. Other means of joining seal assembly 100 to cannula assembly 200 will be readily apparent to one of ordinary skill in the art.

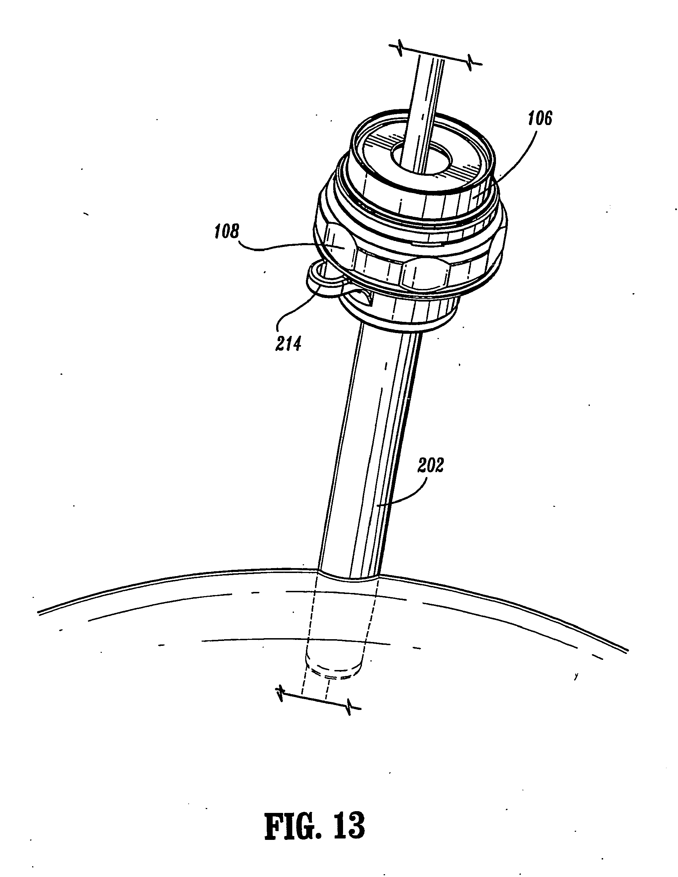

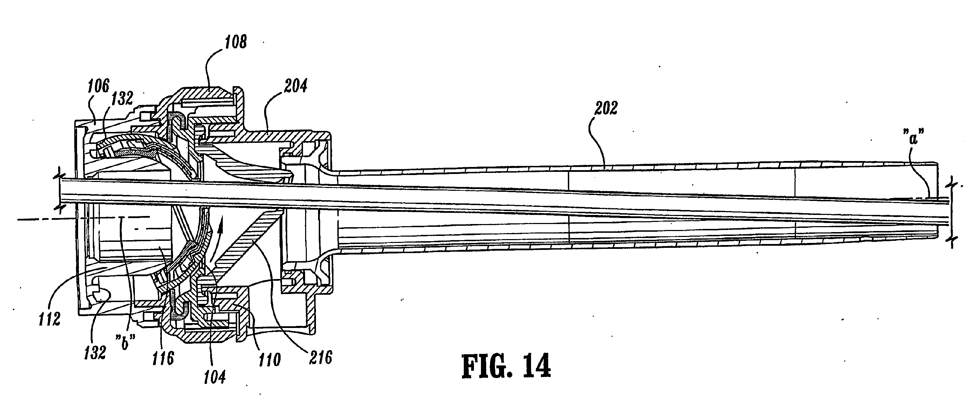

Referring now to FIGS. 13-14, use of the seal assembly 100 and cannula assembly 200 in connection with introduction of a surgical instrument will be discussed. Seal assembly 100 is mounted to cannula assembly 200 which is previously introduced into an insufflated abdominal cavity. An instrument is inserted into seal assembly 100 through passage 116 of inner cylindrical guide wall 112 in seal housing 102. If the axis of the instrument is not perfectly aligned with the axis "a" of cannula assembly 200 or axis "b" of seal assembly 100, then the surgical instrument will contact the inner guide wall 112 and/or the inner surface of seal member 142. Contact with the seal member 142 causes gimbal mount 104 to swivel within seal housing 102, thereby bringing aperture 154 into alignment with the surgical instrument Aperture 154 stretches to accommodate the instrument diameter, as necessary. The instrument passes further distally into the cannula housing 204 passing through duckbill valve 216 and cannula sleeve 202 into the body cavity. As the instrument passes distally, gimbal mount 104 is free to swivel further with respect to seal housing 102. In addition, as the surgeon manipulates the instrument within the body cavity, gimbal mount 104 is free to swivel relative to housing 102, thereby allowing seal member 142 to maintain sealing engagement with the instrument passed therethrough.

While the invention has been particularly shown, and described with reference to the preferred embodiments, it will be understood by those skilled in the art that various modifications and changes in form and detail may be made therein.

## Claims

1. A seal assembly (100) for use with an access device (200), which comprises:
a seal housing (102) defining a central longitudinal axis (b), the seal housing (102) including an inner wall (112) and an outer wall (114), the inner wall defining a longitudinal opening (116) to permit passage of instrumentation through the seal housing; and
a gimbal mount (104) at least partially accommodated within a space (134) defined between the inner wall and the outer wall of the seal housing, the gimbal mount including a seal member (142) defining an aperture (154) for substantial sealed reception of a surgical instrument, the gimbal mount adapted for angular movement relative to the central longitudinal axis upon angulation of the surgical instrument while substantially maintaining the sealed reception of the surgical instrument, **characterised in that:**
the inner wall defines a distal arcuate surface (118) in contacting relation with a corresponding inner arcuate surface of the gimbal mount.

2. The seal assembly according to claim 1 wherein the seal housing includes a skirt seal (132), the skirt seal positioned about the gimbal mount and adapted to minimize passage of fluids through the seal housing.

3. The seal assembly according to claim 2 wherein the skirt seal extends to contact the gimbal mount, the skirt seal adapted to bias the gimbal mount in a general proximal direction.

4. The seal assembly according to claim 3 wherein the skirt seal is dimensioned and configured to bias the gimbal mount against a portion of the inner wall of the seal housing.

5. The seal assembly according to any one of the preceding claims wherein the seal member includes a resilient member and at least one protective layer (150, 152) juxtaposed relative to the resilient member.

6. The seal assembly according to claim 5 wherein the at least one protective layer of the seal member extends at least partially within the aperture to protect portions of the seal member defining the aperture during passage of the surgical instrument.

7. The seal assembly according to claim 5 or 6 wherein the at least one protective layer includes a fabric material.

8. The seal assembly according to any one of the preceding claims wherein the seal housing is adapted to be detachably mounted to a cannula assembly (200) for providing a substantially fluid-tight seal when said instrument is inserted into the seal assembly and through the cannula assembly.

9. The seal assembly according to any one of the preceding claims wherein the gimbal mount defines a general hemispherical configuration.

10. The seal assembly according to any one of the preceding claims wherein the arcuate inner surface of the gimbal mount is in contacting relation with the distal arcuate surface of the seal housing such that the arcuate inner surface traverses the distal arcuate surface upon angular movement of the surgical instrument.

11. The seal assembly according to any one of the preceding claims wherein the seal housing is associated with a cannula assembly (200).

12. The seal assembly according to claim 2 or any one of the preceding claims when dependent on claim 2 wherein the seal assembly includes an upper housing portion (109) and a lower housing portion (110), and the skirt is associated with the lower housing portion.

13. The seal assembly according to claim 12 wherein the skirt seal is disposed on an upper surface of the lower housing portion.

14. The seal assembly according to claim 12 or 13 when dependent on claim 11 wherein the lower housing portion is fixed to the cannula assembly, and the upper housing portion is detachably securable with at least one of the lower housing portion and the cannula assembly.

15. The seal assembly according to any one of the preceding claims wherein the arcuate inner surface of the gimbal mount rides along the distal arcuate surface of the inner wall of the seal housing in contacting relation to permit gimbal mount to swivel within seal housing.

16. The seal assembly of claim 15 wherein the gimbal mount is arranged for swivelling movement about a centre of rotation (c) which is coincident with the central longitudinal axis of the seal housing.

## Patentansprüche

1. Abdichtanordnung (100) zur Verwendung mit einer Zugangsvorrichtung (200), aufweisend:
ein Abdichtgehäuse (102), das eine zentrale Längsachse (b) definiert, wobei das Abdichtgehäuse (102) eine Innenwand (112) und eine Außenwand (114) enthält, wobei die Innenwand eine Längsöffnung (116) definiert, um einen Durchgang von Instrumenten durch das Abdichtgehäuse zu erlauben; und
eine kardanische Aufhängung (104), die zumindest teilweise innerhalb eines Raums (134) aufgenommen ist, der zwischen der Innenwand und der Außenwand des Abdichtgehäuses definiert ist, wobei die kardanische Aufhängung ein Abdichtelement (142) enthält, das eine Öffnung (154) für eine stofflich abgedichtete Aufnahme eines chirurgischen Instruments definiert, wobei die kardanische Aufhängung für eine Winkelbewegung relativ zu der zentralen Längsachse nach einer Winkelbildung des chirurgischen Instruments angepasst ist, während sie im Wesentlichen die abgedichtete Aufnahme des chirurgischen Instruments aufrecht erhält, **dadurch gekennzeichnet, dass**
die Innenwand eine distale gebogene Oberfläche (118) in kontaktierender Beziehung mit einer entsprechenden inneren gebogenen Oberfläche der kardanischen Aufhängung definiert.

2. Abdichtanordnung nach Anspruch 1, wobei das Abdichtgehäuse eine Einfassungsabdichtung (132) enthält, wobei die Einfassungsabdichtung um die kardanische Aufhängung positioniert und angepasst ist, einen Durchgang von Fluiden durch das Abdichtgehäuse zu minimieren.

3. Abdichtanordnung nach Anspruch 2, wobei sich die Einfassungsabdichtung erstreckt, um die kardanische Aufhängung zu kontaktieren, wobei die Einfassungsabdichtung angepasst ist, die kardanische Aufhängung in einer allgemein proximalen Richtung vorzuspannen.

4. Abdichtanordnung nach Anspruch 3, wobei die Einfassungsabdichtung dimensioniert und eingerichtet ist, die kardanische Aufhängung gegen einen Abschnitt der Innenwand des Abdichtgehäuses vorzuspannen.

5. Abdichtanordnung nach einem der vorgehenden Ansprüche, wobei das Abdichtelement ein elastisches Element und zumindest eine Schutzlage (150, 152) enthält, die neben dem elastischen Element angeordnet ist.

6. Abdichtanordnung nach Anspruch 5, wobei sich die zumindest eine Schutzlage des Abdichtelements zumindest teilweise innerhalb der Öffnung erstreckt, um Abschnitte des Abdichtelements zu schützen, welche die Öffnung während des Durchgangs des chirurgischen Instruments definieren.

7. Abdichtanordnung nach Anspruch 5 oder 6, wobei die zumindest eine Schutzlage ein Fasermaterial enthält.

8. Abdichtanordnung nach einem der vorgehenden Ansprüche, wobei das Abdichtgehäuse angepasst ist, um lösbar an einer Kanülenanordnung (200) montiert zu sein, um eine im Wesentlichen fluiddichte Abdichtung zur Verfügung zu stellen, wenn das Instrument in die Abdichtanordnung und durch die Kanülenanordnung eingeführt wird.

9. Abdichtanordnung nach einem der vorgehenden Ansprüche, wobei die kardanische Aufhängung einen allgemein halbkugelförmigen Aufbau definiert.

10. Abdichtanordnung nach einem der vorgehenden Ansprüche, wobei die gebogene innere Oberfläche der kardanischen Aufhängung derart in kontaktierender Beziehung mit der distalen gebogenen Oberfläche des Abdichtgehäuses ist, dass die gebogene innere Oberfläche die distale gebogene Oberfläche nach einer Winkelbewegung des chirurgischen Instruments durchläuft.

11. Abdichtanordnung nach einem der vorgehenden Ansprüche, wobei das Abdichtgehäuse mit einer Kanülenanordnung (200) verbunden ist.

12. Abdichtanordnung nach Anspruch 2 oder einem der vorgehenden Ansprüche in Abhängigkeit von Anspruch 2, wobei die Abdichtanordnung einen oberen Gehäuseabschnitt (109) und einen unteren Gehäuseabschnitt (110) enthält und die Einfassung mit dem unteren Gehäuseabschnitt verbunden ist.

13. Abdichtanordnung nach Anspruch 12, wobei die Einfassungsabdichtung an einer oberen Oberfläche des unteren Gehäuseabschnitts angeordnet ist.

14. Abdichtanordnung nach Anspruch 12 oder 13 in Abhängigkeit von Anspruch 11, wobei der untere Gehäuseabschnitt an der Kanülenanordnung befestigt ist und der obere Gehäuseabschnitt abnehmbar an dem unteren Gehäuseabschnitt und/oder der Kanülenanordnung befestigbar ist.

15. Abdichtanordnung nach einem der vorgehenden Ansprüche, wobei die gebogene innere Oberfläche der kardanischen Aufhängung in kontaktierender Beziehung entlang der distalen gebogenen Oberfläche der Innenwand des Abdichtgehäuses gleitet, um es der kardanischen Aufhängung zu erlauben, innerhalb des Abdichtgehäuses zu schwenken.

16. Abdichtanordnung nach Anspruch 15, wobei die kardanische Aufhängung für eine Schwenkbewegung um einen Rotationsmittelpunkt (c) angeordnet ist, der mit der zentralen Längsachse des Abdichtgehäuses zusammenfällt.

## Revendications

1. Ensemble d'étanchéité (100) pour utilisation avec un dispositif d'accès (200), qui comprend:
un boîtier d'étanchéité (102) définissant un axe longitudinal central (b), le boîtier d'étanchéité (102) comprenant une paroi interne (112) et une paroi externe (114), la paroi interne définissant une ouverture longitudinale (116) pour permettre le passage d'instruments à travers le boîtier d'étanchéité; et
une suspension à cardan (104) au moins partiellement logée dans un espace (134) défini entre la paroi interne et la paroi externe du boîtier d'étanchéité, la suspension à cardan comportant un élément d'étanchéité (142) définissant une ouverture (154) pour une réception sensiblement étanche d'un instrument chirurgical, la suspension à cardan étant conçue pour un mouvement angulaire relativement à l'axe longitudinal central lors de l'angulation de l'instrument chirurgical tout en maintenant sensiblement la réception étanche de l'instrument chirurgical, **caractérisé en ce que:**
la paroi interne définit une surface distale arquée (118) en relation de contact avec une surface interne arquée correspondante de la suspension à cardan.

2. Ensemble d'étanchéité selon la revendication 1, dans lequel le boîtier d'étanchéité comprend un joint d'étanchéité à collerette (132), le joint d'étanchéité à collerette étant positionné autour de la suspension à cardan et conçu pour réduire à un minimum le passage de fluides à travers le boîtier d'étanchéité.

3. Ensemble d'étanchéité selon la revendication 2, dans laquelle le joint d'étanchéité à collerette s'étend pour venir en contact avec la suspension à cardan, le joint d'étanchéité à collerette étant apte à solliciter la suspension à cardan dans une direction générale proximale.

4. Ensemble d'étanchéité selon la revendication 3, dans lequel le joint d'étanchéité à collerette est dimensionné et configuré pour solliciter la suspension à cardan contre une portion de la paroi interne du boîtier d'étanchéité.

5. Ensemble d'étanchéité selon l'une quelconque des revendications précédentes, dans lequel l'élément d'étanchéité comporte un élément résiliant et au moins une couche de protection (150, 152) juxtaposée relativement à l'élément résiliant.

6. Ensemble d'étanchéité selon la revendication 5, dans lequel la au moins une couche de protection de l'élément d'étanchéité s'étend au moins partiellement dans l'ouverture pour protéger des portions de l'élément d'étanchéité définissant l'ouverture durant le passage de l'instrument chirurgical.

7. Ensemble d'étanchéité selon la revendication 5 ou 6, dans lequel la au moins une couche de protection comporte un matériau d'étoffe.

8. Ensemble d'étanchéité selon l'une quelconque des revendications précédentes, dans lequel le boîtier d'étanchéité est conçu pour être monté amoviblement sur un ensemble de canule (200) afin de réaliser un joint sensiblement étanche au fluide lorsque ledit instrument est inséré dans l'ensemble d'étanchéité et à travers l'ensemble de canule.

9. Ensemble d'étanchéité selon l'une quelconque des revendications précédentes, dans lequel la suspension à cardan définit une configuration générale hémisphérique.

10. Ensemble d'étanchéité selon l'une quelconque des revendications précédentes, dans lequel la surface interne arquée de la suspension à cardan est en relation de contact avec la surface distale arquée du boîtier d'étanchéité de sorte que la surface interne arquée traverse la surface distale arquée lors d'un mouvement angulaire de l'instrument chirurgical.

11. Ensemble d'étanchéité selon l'une quelconque des revendications précédentes, dans lequel le boîtier d'étanchéité est associé à un ensemble de canule (200).

12. Ensemble d'étanchéité selon la revendication 2 ou l'une quelconque des revendications précédentes, dépendant de la revendication 2, dans lequel l'ensemble d'étanchéité comporte une portion de boîtier supérieure (109) et une portion de boîtier inférieure (110), et la collerette est associée à la portion de boîtier inférieure.

13. Ensemble d'étanchéité selon la revendication 12, dans lequel le joint d'étanchéité à collerette est disposé sur une surface supérieure de la portion de boîtier inférieure.

14. Ensemble d'étanchéité selon la revendication 12 ou 13, dépendant de la revendication 11, dans lequel la portion de boîtier inférieure est fixée à l'ensemble de canule, et la portion de boîtier supérieure peut être fixée amoviblement à au moins une parmi la portion de boîtier inférieure et l'ensemble de canule.

15. Ensemble d'étanchéité selon l'une quelconque des revendications précédentes, dans lequel la surface interne arquée de la suspension à cardan se déplace le long de la surface distale arquée de la paroi interne du boîtier d'étanchéité en une relation de contact pour permettre à la suspension à cardan de pivoter dans le boîtier d'étanchéité.

16. Ensemble d'étanchéité selon la revendication 15, dans lequel la suspension à cardan est agencée en vue d'un mouvement de pivotement autour d'un centre de rotation (c) qui coïncide avec l'axe longitudinal central du boîtier d'étanchéité.
